# EUROPEAN PATENT SPECIFICATION

(11) **EP 3 958 793 B1**
(45) Date of publication and mention of the grant of the patent: **28.01.2026**
(21) Application number: 20724674.5
(22) Date of filing: 17.04.2020
(51) Int. Cl.: A61F 2/24, A61M 25/01, A61M 25/00

(54) **EXPANDABLE SHEATH**
DEHNBARE HÜLLE
GAINE EXTENSIBLE

(30) Priority: 22.04.2019 US 201962837060 P; 19.07.2019 US 201962876466 P
(43) Date of publication of application: 02.03.2022
(60) Divisional application: 25222924.0
(73) Proprietor: W. L. Gore & Associates, Inc., Newark, DE 19711 (US)
(72) Inventor: BROYLES, Michael R., Newark, Delaware 19711 (US); HAARER, Joshua C., Newark, Delaware 19711 (US); HOUGE, Reed A., Newark, Delaware 19711 (US); SHAW, Edward E., Newark, Delaware 19711 (US)
(74) Representative: HGF
(86) International application number: PCT/US2020/028765
(87) International publication number: WO 2020/219363

(56) References cited:
- EP-A2- 1 819 391
- US-A1- 2009 240 202
- US-A1- 2012 083 877
- US-A1- 2016 128 723
- US-A1- 2017 281 344
- US-A1- 2018 008 407

## Description

### CROSS-REFERENCE TO RELATED APPLICATION

This application claims the benefit of Provisional Application No. 62/837,060, filed April 22, 2019, and also claims the benefit of Provisional Application No. 62/876, 466, filed July 19, 2019.

### FIELD

The present disclosure relates to introducer sheaths for medical procedures including delivering an endoprosthesis and other medical devices to a treatment region in the vasculature of a patient.

### BACKGROUND

Current methods for providing medical treatment to human vasculature involve the use of catheters and endovascularly deliverable medical devices. Non-limiting examples include the endovascular delivery of endoprostheses, such as, for example, stents and stent grafts (self-expanding or otherwise), bifurcated stents and stent grafts, drug-eluting stents, and vascular filters, as well as endoluminal imaging devices.

Such catheters and other medical devices sometimes enter the body through an orifice or incision. In some instances, a medical conduit is inserted through the orifice or incision, and the catheter and other medical devices are passed through the medical conduit. Such medical conduits are sometimes referred to as introducer sheaths. Advancements continue to be made to minimize the size of the incision required to accommodate the catheter or other medical device being advanced into the body, as well as to minimize the trauma to the patient as the catheter or medical device is advanced through the patient's vasculature. However, in some cases, relatively large objects must be inserted through the incision and advanced through the vasculature to the treatment site to effectively treat a patient's condition. In such instances, while it may not be possible to reduce a profile or cross section of the medical device, it may be possible to configure the introducer sheath to help minimize the trauma inflicted to the patient's anatomy during the medical procedure. US2012083877A1 discloses a sheath for use with catheter assembly to introduce e.g. mitral valve, into patient's heart. The sheath has a folded portion between overlapping and underlying portions, where portion of sheath is arranged to self expand to accommodate prosthetic device

### CROSS-REFERENCE TO RELATED APPLICATION

This application claims the benefit of Provisional Application No. 62/837,060, filed April 22, 2019, and also claims the benefit of Provisional Application No. 62/876,466, filed July 19, 2019,

### DEFINITIONS AND TERMINOLOGY

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

As used herein, the term "controlled retraction" refers to causing articles to shorten in length in at least one direction by the application of heat, by wetting with a solvent, or by any other suitable means or combinations thereof in such a way as to inhibit folding, pleating, or wrinkling of the subsequent article visible to the naked eye.

As used herein, the term "elastic" refers to the ability of a material or article to resume or return to its approximate original dimensions spontaneously after contraction, dilatation, elongation, or other distortion upon removal of a contraction, dilation, elongation or other distortion force. The term "inelastic" as used herein refers to the inability of a material or article to resume or return to its approximate original dimensions spontaneously after contraction, dilatation, elongation, or other distortion upon removal of a contraction, dilation, elongation or other distortion force.

As used herein in the context of a texture, or surface feature, the term "smooth" means free of wrinkles, folds, creases, pleats and similar surface features that would be visible to the naked eye unaided by magnification along a particular portion or area of a surface. A "majority" of a surface of an article (e.g., an inner surface or outer surface of a tubular member) is smooth when more than 50% of the surface is smooth. "Substantially all" of a surface of an article (e.g., an inner surface or outer surface) is smooth when 90% or more of the surface is smooth. And, an "entire surface" of an article (e.g., an inner surface or outer surface) is smooth when the entire surface is smooth. As a non-limiting example, a majority of a surface is smooth where more than 50% of the surface as measured along a major dimension (e.g., length) of the article is smooth (e.g., a tubular member that is 10 cm long is wrinkle free along more than 5 cm of the length of the tubular member). Similarly, and as another non-limiting example, substantially all of the surface of an article is smooth where 90% or more of the surface as measured along a major dimension (e.g., length) of the article is smooth (e.g., a tubular member that is 10 cm long is wrinkle free long at least 9 cm of the length of the tubular member).

For purposes of this disclosure, a component is considered to be "wrinkle-free" if within a 1 cm length of the component, the surface of the component is devoid of wrinkles, folds, creases, pleats and similar surface features that would be visible to the naked eye unaided by magnification. It is to be noted that the terms "free of folds," "devoid of folds," and "fold free" are used interchangeably herein.

### BRIEF DESCRIPTION OF THE DRAWINGS

The accompanying drawings are included to provide a further understanding of the present disclosure and are incorporated in and constitute a part of this specification, illustrate embodiments described herein, and together with the description serve to explain the principles discussed in this disclosure.
FIG. 1 is an illustration of a medical system including an expandable introducer sheath in accordance with an embodiment;
FIG. 2 is a detailed view of a distal end of the expandable introducer sheath of the medical system of FIG. 1 in accordance with an embodiment;
FIG. 3 is a detailed view of a distal end of the expandable introducer sheath of the medical system of FIG. 1 in accordance with an embodiment;
FIG 3A is a cross-sectional view of the expandable introducer sheath of FIG. 3 taken along line 3A-3A;
FIG. 4 is an illustration of a support framework of an expandable introducer sheath in accordance with an embodiment;
FIG. 5 is an illustration of a support framework of an expandable introducer sheath in accordance with an embodiment;
FIG. 6 is an illustration of a medical system including an expandable introducer sheath that is partially expanded in accordance with an embodiment;
FIG. 7 is an illustration of a medical apparatus including an inline sheath dilator placed over a delivery catheter which carries a medical device attached thereto proximate a distal end portion of the delivery catheter, in accordance with an embodiment;
FIG. 8 is an illustration of the medical apparatus of FIG. 7, with the medical device in a compacted configuration;
FIG. 9 is an illustration of the medical apparatus of FIG. 8, with the inline sheath dilator disposed over the medical device;
FIG. 10 is an illustration of a medical apparatus including an expandable sheath disposed along a guidewire with the assistance of a dilator, in accordance with an embodiment;
FIG. 11 is an illustration of the medical apparatus of FIG. 10 with the dilator removed;
FIG. 12 is an illustration of the medical apparatus of FIG. 11, with the medical apparatus of FIG. shown 9 advancing toward the expandable sheath along the guidewire;
FIG. 13 is an illustration of the medical apparatus of FIG. 12, with the inline sheath dilator inserted into the expandable sheath to expand the expandable sheath;
FIG. 14 is an illustration of the medical apparatus of FIG. 13, with the delivery catheter extended from the inline sheath dilator;
FIG. 15 is an illustration of the medical apparatus of FIG. 14 in accordance with one embodiment, shown with the distal end portion of the delivery catheter carrying the medical device positioned at a target site in the body of a patient;
FIG. 16 is an illustration of the medical apparatus of FIG. 14 following medical device deployment;
FIG. 17 is an illustration of the medical apparatus of FIG. 16, with the delivery catheter retracted into the inline sheath dilator;
FIG. 18 is an illustration of the medical apparatus of FIG. 17, with the inline sheath dilator retracted from the expandable sheath;
FIG. 19 is an illustration of the medical apparatus of FIG. 18, following retraction of the expandable sheath from the body of the patient, according to one embodiment.
FIGs. 20A and 20B are illustrations of an expandable sheath with an inelastic lubricious liner in pleated and unpleated configurations in accordance with an embodiment;
FIGs. 21A and 21B are illustrations of an expandable sheath with an inelastic lubricious liner in pleated and unpleated configurations in accordance with an embodiment;
FIGs. 22A and 22B are illustrations of an expandable sheath in compressed and expanded configurations in accordance with an embodiment.

### DETAILED DESCRIPTION

Persons skilled in the art will readily appreciate that various aspects of the present disclosure can be realized by any number of methods and apparatus configured to perform the intended functions. Stated differently, other methods and apparatuses can be incorporated herein to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Embodiments herein include various apparatuses, systems, and methods for expandable introducer sheaths, such as, but not limited to, introducer sheaths for use in association with medical procedures, such as endovascular procedures. As such, embodiments discussed herein comprise an endovascular introducer sheath for providing minimally invasive access to various regions of the patient's vasculature, such as the aorta. The introducer sheath is generally tubular and includes a proximal end, a distal end, and a central through lumen. The introducer sheath is configured such that it can be expanded in situ (e.g., while extending within a patient's anatomy), and such that it is adapted to contract (e.g., self-recover) in situ after being expanded. For instance, in various embodiments, the introducer sheath is adapted to be expanded from an initial diameter to a larger expanded diameter (also referred to herein as an expanded diameter) to accommodate the passing of one or more medical devices through the lumen of the introducer sheath. The introducer sheath is also adapted such that the introducer sheath can self-recover from the larger expanded diameter to a diameter less than the larger expanded diameter. That is, in various embodiments, the introducer sheath of the present disclosure is adapted to self-recover from a large expanded diameter to a smaller diameter without requiring an external influential force for such recovery.

In various examples, the introducer sheath is expandable in circumference in response to outward pressure applied from within the lumen of the introducer sheath. By expanding and contracting as such, the introducer sheath of the present disclosure is operable to maintain a reduced profile or cross-sectional area, and thereby minimize trauma to the surrounding anatomy within which it is situated. The operation of the introducer sheath of the present disclosure is also such that the introducer sheath functions as a bearing surface between the medical device and the patient's anatomy, such as the wall of the patient's vessel within which the introducer sheath is situated. As such a bearing surface, the medical device(s) being passed through the lumen of the introducer sheath do not directly contact the vessel wall, for example. While some convention introducer sheaths may be configured to unfold to a larger diameter, for example, such conventional devices do not recover to a smaller diameter than the unfolded larger diameter after being expanded.

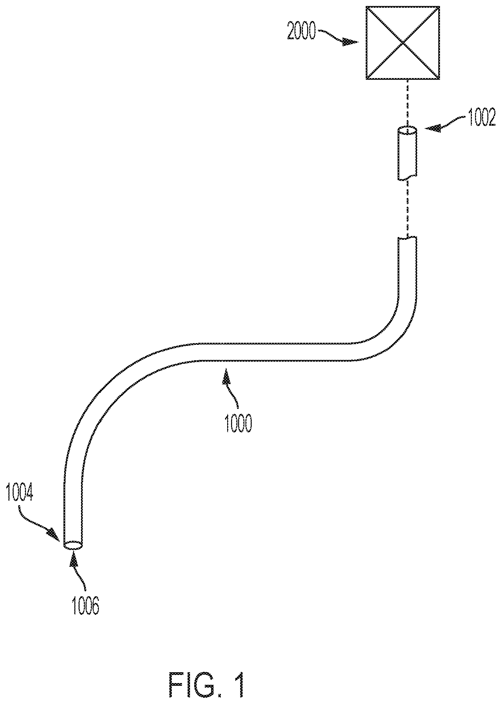

FIG. 1 is a perspective view of a medical system 10. As shown, the medical system 10 includes an introducer sheath 1000. In various examples, the medical system 10 optionally includes one or more auxiliary components 2000, which may include, but are not limited to, control handles, valves, such as hemostatic valves, hubs configured to facilitate the passage of instrumentation, and connectors, including luer fittings and others used in controlling fluid backflow through one or more components of the medical system 10 (e.g., Tuohy-Borst Connector(s)).

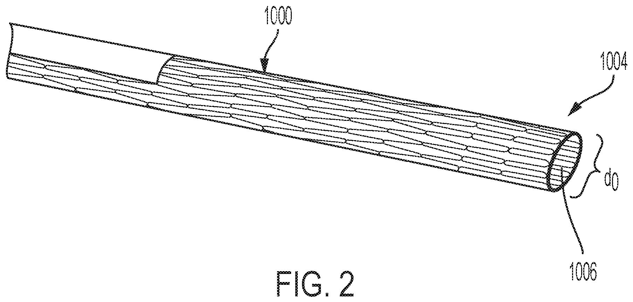

The introducer sheath 1000 of the medical system 10 is an elongate element having a proximal end 1002, a distal end 1004, and a lumen 1006. The lumen 1006 extend through the introducer sheath 1000 from the proximal end 1002 to the distal end 1004 such that the proximal and distal ends 1002 and 1004 are each open to the lumen 1006. FIG. 2 is a detailed view of a distal portion of the introducer sheath 1000, showing the lumen 1006 open at the distal end 1004 of the introducer sheath 1000. In various embodiments, the lumen 1006 is configured to facilitate the passing of one or more medical devices through the introducer sheath 1000. As such, the lumen 1006 is configured to accommodate such medical devices. In various examples, as discussed further below, the introducer sheath 1000 is configured such that a diameter of the lumen 1006 can be expanded to accommodate such medical devices. In some examples, the lumen 1006 is configured to progressively expand along the length of the introducer sheath 1000 to accommodate medical devices as they are advanced distally through the lumen 1006. This progressive expansion of the lumen 1006 may be continuous or step-wise, as discussed further below. For example, the introducer sheath 1000 may have an introduction outside diameter that ranges from 3 to 20 French (Fr), and a working length ranging between 40cm and 200cm. For instance, the introducer sheath 1000 may have a working length of 75cm to 150cm. In some examples, the introduction outside diameter may be 5Fr to 10Fr. In some such examples, the introducer sheath 1000 is configured to expand to permit instruments ranging up to 30Fr to pass through the lumen 1006 of the introducer sheath 1000. As such, it will be appreciated that the outside diameter of the introducer sheath 1000 will be expanded to a diameter in excess of 30Fr under such conditions. In some examples, the introducer sheath 1000 may be expandable to an outside diameter of 3Fr to 30Fr or greater.

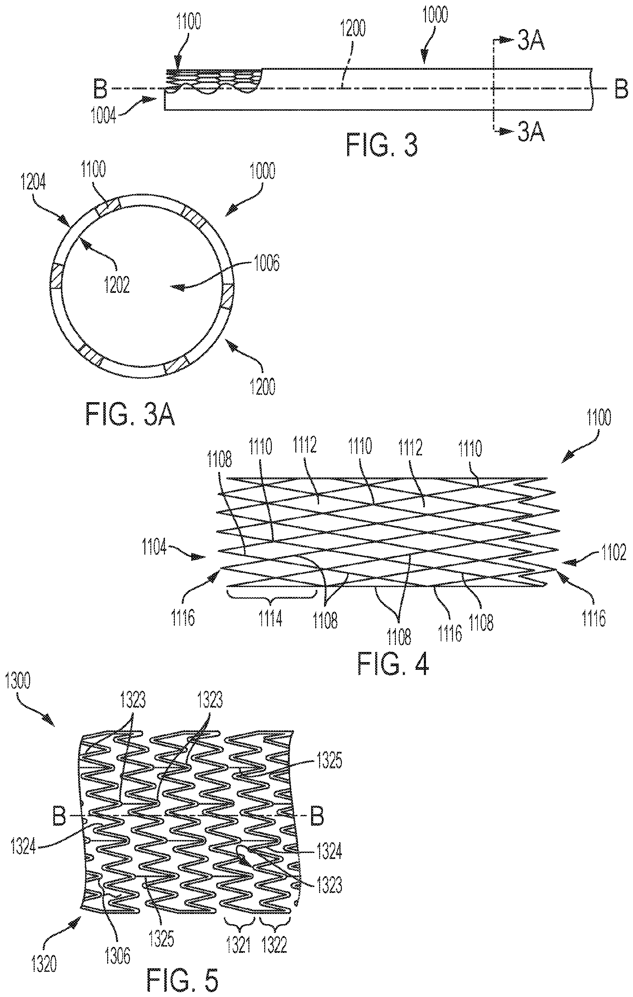

Turning now to FIG. 3, the introducer sheath 1000 generally includes a support framework 1100 and a covering 1200. The support framework 1100 is adapted to support the covering 1200 and provide one or more of axial compressive strength and hoop strength to the introducer sheath 1000. In some examples, the support framework 1100 operates to minimize a kinkability of the introducer sheath 1000. For instance, in some examples, a kinkability of the support framework is less than a kinkability of the covering 1200.

The covering 1200 is configured to isolate the lumen 1006 of the introducer sheath 1000 and help define, at least in part, a barrier between an interior of the introducer sheath 1000 and an exterior of the introducer sheath 1000. In some examples, the covering 1200 helps isolate the support framework 1100 from one or more of the patient's vasculature, and the medical devices advanced through the lumen 1006 of the introducer sheath 1000. Such isolation helps minimize a potential for medical device(s) being advanced through the lumen 1006 of the introducer sheath 1000 from becoming entangled with the support framework 1100. In some examples, the covering 1200 may optionally define a lubricious layer that helps reduce friction between the introducer sheath 1000 and one or more of the patient's vasculature, and the medical devices advanced through the lumen 1006 of the introducer sheath 1000.

FIG. 4 provides an example illustration of the support framework 1100. The support framework 1100 may be configured, in combination with the covering 1200, to cause the introducer sheath 1000 to adopt an initial profile or configuration where the lumen 1006 of the introducer sheath 1000 has an initial diameter when no external forces act upon the introducer sheath 1000. In such examples, the initial exterior diameter of the introducer sheath 1000 is generally one that is configured for insertion into the patient's vasculature (e.g., between 3Fr and 20Fr).

The support framework 1100 is also configured such that it is expandable to facilitate expansion of one or more portions of the introducer sheath 1000 from the initial configuration to an expanded configuration where the lumen of the introducer sheath 1000 has a diameter that exceeds the initial diameter. These initial or contracted configurations and expanded or enlarged configurations can include various shapes, including circular profiles and non-circular profiles (such as ovals, for example). The support framework 1100 can be formed from an elastic or springy material that allows the framework to be resiliently deformed in accordance with the initial and expanded configurations referred to above.

As shown, the support framework 1100 generally includes a tubular shaped member having a proximal end 1102 and a distal end 1104, and a through lumen 1106. The support framework includes a plurality of strut elements 1108 situated between the proximal and distal ends 1102 and 1104. In various examples, one or more of the strut elements 1108 intersect with one another at intersection points or nodes 1110. As shown, the interconnected strut elements 1108 define one or more closed cells 1112. In some examples, the closed cells are diamond shaped, though other shapes are contemplated. In some examples, adjacent closed cells 1112 share a common intersection point or node 1110 as shown in FIG. 4. In some examples, the intersection points or nodes 1110 at the proximal and distal ends of each of the respective cells define apices 1116.

In some examples, the closed cells 1112 may define one or more rows 1114 of cells. Where the support framework 1100 includes a plurality of rows of closed cells 1112, adjacent rows (e.g., longitudinally adjacent rows) of closed cells may be coupled to one another, either directly or indirectly. Direct coupling between adjacent rows 1114 generally occurs at the apices 1116 where the adjacent rows 1114 intersect with one another. Indirect coupling may include one or more longitudinal connectors that operate to couple adjacent rows 1114 together. In various examples, such longitudinal connectors may extend between apices of adjacent rows 1114, or may extend between other portions of the strut elements 1108, such as a mid-point between ends of a strut element 1108.

In some examples, in addition to or as an alternative to the closed cells 1112, the interconnected struts may define one or more circumferential members and/or helical elements that have undulations, as shown in FIG. 5. The undulations may be formed by struts interconnected at bends or apices, and may be arranged into wave-like configurations. The undulations can form various patterns, such as sinusoidal patterns, zigzag patterns or similar geometric patterns. The undulations of the helical element can form a series of rows or turns along the length of the support framework.

FIG. 5 depicts a support framework 1300 with a cylindrical body 1302 according to one embodiment. The support framework 1300 can be made in various forms including various lengths and inside diameters.

A continuous pattern of undulations 1306 forms a series of helical turns about the longitudinal axis along line B-B, shown in FIGs. 3 and 5. Helical turns 1321, 1322 can form a substantially cylindrical, tubular helical element 1320. The helical turns 1321, 1322 have a number of apices 1323. These apices 1323 are formed where two or more struts 1324 interconnect. In FIG. 5, the helical element 1320 may be axially interposed between and directly connected to a proximal and distal row of closed cells (see, e.g., FIG. 4). Various connecting struts 1325 can be provided to contribute to longitudinal stability to the stent. For example, these connecting struts or connectors can join adjacent structures, turns or rows of the support framework 1300 as similarly described above with reference to support framework 1100.

The support framework (e.g., 1100 and/or 1300) can be formed from a wide variety of materials or combinations of materials, including metals and plastics. Among metals that can be used are stainless steel, titanium, tantalum, alloys such as Elgiloy^{®} and Phynox^{®} spring alloys, 316 stainless steel, MP35N^{®} alloy, and Nitinol nickel-titanium alloy. Super-elastic versions or shape memory versions of the mentioned alloys can also be used. Use of nitinol alloys can impart biasing characteristics to the support framework. In such a nitinol support framework, the phase behavior of the material can be selected, and the support framework treated so that the support framework has a tendency to influence the introducer sheath 1000 to adopt the initial configuration, one or more expanded configurations, or some configuration therebetween. Suitable plastics may include PEEK, PEBAX, PTFE, Nylon, Polyethylene, and others.

In various examples, any technique that produces a support framework (e.g., 1100 and/or 1300) with the required characteristics can be used. For example, the support framework can be cut from a continuous tube of material (e.g., nitinol) into the desired pattern, such as through use of a laser. In some such examples, therefore, the support framework forms a monolithic construct or unibody. The support framework can also be constructed by various known techniques such as machining, chemical etching, laser ablation, die-cutting, plasma etching, stamping, water jet cutting or any other suitable means as long as the required support framework and associated material properties can be achieved. The support framework can also be formed from a flat sheet of material that is cut into the desired pattern and then bonded together to form a tube having a seam. Finally, the support framework can be constructed from wires or ribbons that are formed into the desired shapes and then bonded together, for example by welding, into the final pattern.

As mentioned above, in various embodiments, the introducer sheath 1000 includes a covering 1200. The covering 1200 can be provided on the interior or exterior surfaces of the support framework 1100, or both as shown in FIG. 2. In various examples, the covering 1200 generally includes a membrane that is reinforced with one or more materials to provide the covering 1200 with elastomeric properties. Such elastomeric properties provide that the covering 1200 is adapted to elastically stretch in accordance with an expansion of the introducer sheath 1000. In some examples, the membrane is porous or otherwise includes a plurality of void spaces. In some examples, the covering 1200 includes one layer of reinforced membrane and another layer without the reinforcement.

Suitable membrane materials for the covering 1200 include, but are not limited to, polymers such as olefin, PEEK, polyamide, polyurethane, polyester, such as polyethylene terephthalate (PET), polyethylene, polypropylene, polyurethane, silicone, fluorinated ethylene propylene (FEP), polytetrafluoroethylene (PTFE), expanded polytetrafluoroethylene (ePTFE), and fluoroelastomers, such as tetrafluoroethylene/polymethylvinylether (TFE/PMVE) copolymers. In a preferred embodiment, the membrane of the covering 1200 may be anisotropic such that it is highly oriented in the one direction.

Membrane material composites may be incorporated that result in articles (e.g., tubular forms) that are relatively free from infolding at both an initial, smaller diameter and an expanded, larger diameter. Such materials can be configured to exhibit high degrees of elongation (longitudinally and/or radially). Such high elongation capabilities can be facilitated by forming relatively straight fibrils into serpentine fibrils (or otherwise bent or compressed fibrils) that substantially straighten upon the application of a force in a direction opposite to a compressed direction. The creation of the serpentine fibrils can be achieved through a thermally induced, controlled retraction of an expanded polytetrafluoroethylene (ePTFE) membrane, through wetting the membrane with a solvent, such as, but not limited to, isopropyl alcohol or Fluorinert^{®} (a perfluorinated solvent commercially available from 3M, Inc., St. Paul, Minn.), or by a combination of such techniques. The retraction of the article does not result in visible pleating, folding, or wrinkling of the ePTFE, unlike what occurs during mechanical compression. Such retraction can be applied to very thin membranes and the membranes may then be combined with one or more other materials (e.g., an elastomer and/or lubricious material) to form a composite membrane having a high degree of extensibility from an initial, smaller diameter that is smooth (e.g., with little to no wrinkling, folds, pleats or the like on a majority, substantial all, or an entirety of a surface), which is also smooth at the expanded, larger diameter. Additional examples of retracted membranes and associated formation techniques may be found in US Patent Application Publication No. 2018/0177583 filed February 22, 2018 to Cully et al.

Suitable reinforcing materials include, but are not limited to, aromatic and aliphatic polyurethanes including copolymers, styrene block copolymers, silicones, thermoplastic elastomers, fluoro-silicones, fluoroelastomers, THV and latex. In some examples, the reinforcing materials include aromatic polyester-based thermoplastic polyurethanes (TPUs) such as Tecothane^{™}.

In various examples, the membrane of the covering 1200 may be reinforced with the reinforcing material by imbibing or other known processes to form the covering 1200 having elastomeric properties. In various examples, the covering 1200 may be blood impermeable such that the introducer sheath 1000 is blood impermeable (even where the support framework is otherwise permeable to blood). Such a configuration provides that leakage of blood from the introducer sheath 1000 can be minimized. In some examples, the covering 1200 forms a lubricious and/or impermeable portion (e.g., one or more layers) of the sheath 1000.

The reinforcing material can be applied to the membrane of the covering 1200 through any number of conventional methods including, but not limited to, lamination, transfer roll coating, wire-wound bar coating, reverse roll coating, solution coating, or other known processes that operate to at least partially fill a portion of the pores of the membrane material with the reinforcing material (e.g., imbibing). For instance, in some examples, the reinforcing material is solution imbibed into the membrane material of the covering 1200. In some such examples, the continuous polymer layer is dissolved in a suitable solvent and coated onto and throughout the membrane material using a wire-wound rod process. The coated membrane material is then passed through a solvent oven and the solvent is removed leaving a continuous polymer layer of reinforcing material coated onto and throughout the membrane material. As such, in some examples, the reinforcing material may be imbibed into the membrane material such that the imbibed reinforcing material fills the pores of the membrane material.

In some cases, such as when silicone is used as the reinforcing material, the coated membrane material may not require the removal of solvent. In another embodiment, the reinforcing material is coated onto at least one side of the membrane material and then subsequently cured. For example, an ultraviolet light (UV) curable urethane may applied to the membrane material and subsequently exposed to UV light and cured to form the covering 1200.

The reinforcing material may be coated onto one or both sides of the membrane material to form the covering 1200. The membrane and/or the reinforcing material may be configured such that the covering 1200 forms a lubricious layer of the introducer sheath 1000. Such a lubricious layer operates to reduce friction between the introducer sheath 1000 and one or more of the medical device(s) and the vasculature.

In various examples, the covering 1200 can be joined to the support framework (e.g., 1100 and/or 1300) over all or over only a portion of the length of the support framework (e.g., 1100 and/or 1300). The covering 1200 can be joined intermittently (e.g., at the proximal and distal ends of the support framework, at one or more nodes 1110 of the strut elements 1108, and/or along one or more of the strut elements 1108). Alternatively, the covering 1200 can be joined to substantially all outwardly and/or inwardly facing portions of the strut elements 1108. Thus, it is to be appreciated that the covering 1200 can be on the outside of the framework (e.g., 1100 and/or 1300), on the inside of the framework (e.g., 1100 and/or 1300), or it can be on both. In some examples where the covering 1200 extends along both the inside and outside of the framework (e.g., 1100 and/or 1300), the covering 1200 may include a continuous tube that is everted over itself to form an interior portion 1202 and an exterior portion 1204 with the framework (e.g., 1100 and/or 1300) situated therebetween (see, e.g., FIG. 3A). In some examples, the interior and exterior portions 1202 and 1204 may be coupled together in accordance with the manufacturing processes disclosed herein. In some such examples, the covering 1200 may be everted (and thus include a transition between the interior and exterior portions) at either of the proximal and distal ends 1002 and 1004 of the introducer sheath 1000 such that a distance between the proximal and distal ends 1002 and 1004 defines an axial length of the introducer sheath 1000. In some examples where the covering 1200 is everted and extends along both the outside and the inside of the framework (e.g., 1100 and/or 1300) the free ends of the covering 1200 (e.g., the ends opposite the transition) can be coupled to one another such that the covering 1200 is substantially continuous (e.g., does not include gaps that otherwise expose the support framework). In some examples, the covering 1200 may additionally or alternatively include or be formed from one or more wraps or windings of the material forming the covering 1200 described herein about one or more portions of the support framework (e.g., 1100 and/or 1300).

In some examples, attachment of the covering 1200 to the framework (e.g., 1100 and/or 1300) can be accomplished by mechanical means such as fiber, adhesive, or discrete mechanical attachment points (clips, etc.). These components also can be bonded together through heat treatment (such as, sintering of the materials together) or through use of a wrap (for instance a tube, tape, or membrane) around the outside of the framework (e.g., 1100 and/or 1300) and covering 1200 (either continuous or discontinuous), that is adhered through either a thermoplastic or thermoset adhesive. Combinations of these methods also can be used.

Suitable biocompatible adhesives include thermoplastic adhesives such as fluorinated ethylene propylene (FEP), polyurethane, cyanoacrylate, thermoplastic fluoropolymer, including fluoroelastomers. Thermoset adhesives are also useful, such as urethanes or silicones including room temperature vulcanizing (RTV) silicone.

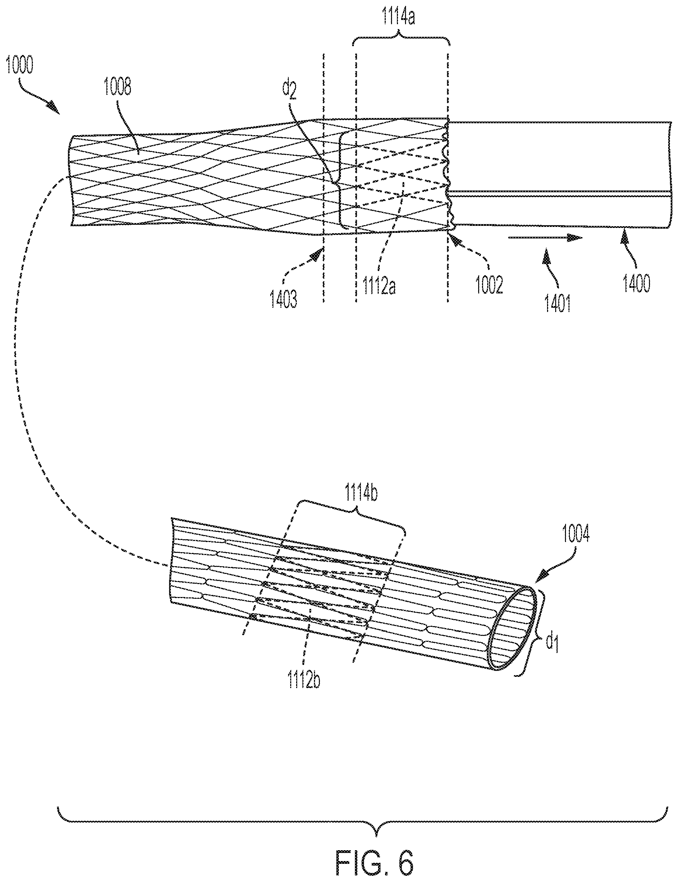

With reference now to FIGS. 1 and 6, the introducer sheath 1000 is operable to expand and contract to facilitate delivery of one or more medical devices through the lumen 1006 of the introducer sheath 1000. FIG. 6 provides an illustration of the introducer sheath 1000 having a medical device 1400 extending from the lumen 1006 of the introducer sheath 1000 at the proximal end 1002 of the introducer sheath 1000. As shown, the proximal end 1002 of the introducer sheath 1000 is expanded (e.g., diameter d₂) relative to the distal end 1004 of the introducer sheath 1000 (e.g., post-delivery diameter d₁). Likewise, the proximal end 1002 of the introducer sheath 1000 is expanded relative to an intermediate portion 1008 of the introducer sheath 1000 between the proximal and distal ends 1002 and 1004. This expansion of the proximal end 1002 of the introducer sheath 1000 is in response to the profile of the medical device 1400 exceeding the initial diameter (e.g., FIG. 2 d₀).

In this illustrated example of FIG. 6, it is to be appreciated that the medical device 1400 is in the process of being proximally withdrawn from the lumen 1006 of the introducer sheath 1000 in the direction of arrow 1401 after having been advanced through the lumen 1006 of the introducer sheath 1000 and distally out of the distal end 1004 of the introducer sheath 1000. As such, it is to be appreciated that the intermediate portion 1008 and distal end 1004 of the introducer sheath 1000 have each contracted from the expanded diameter d₂ to a post-delivery diameter, shown as d₁. That is, while the intermediate portion 1008 and the distal end 1004 were previously expanded in diameter to accommodate the profile of the medical device 1400, each of the intermediate portion 1008 and the distal end 1004 have contracted to the post-delivery diameter d₁ as a result of the distal end 1403 of the medical device 1400 being proximal to each of the intermediate portion 1008 and the distal end 1004.

As shown in FIG. 6, the closed cells in the expanded region of the introducer sheath 1000 (e.g., closed cells 1112a of row 1114a shown in broken lines) are expanded relative to the closed cells in non-expanded regions of the introducer sheath 1000 (e.g., closed cells 1112b if row 1114b shown in broken lines).

As mentioned above, in various embodiments, the introducer sheath 1000 is configured such that the introducer sheath 1000 can self-recover from the larger expanded diameter to a diameter less than the larger expanded diameter, such as without requiring an external influential force for such recovery. As such, it is to be appreciated that this expansion and contraction of the introducer sheath 1000 may be progressive along the length of the introducer sheath 1000, and thus is not configured such that the entire length of the introducer sheath 1000 must be in either an expanded configuration or a contracted/initial configuration. Instead, it is to be appreciated the introducer sheath 1000 is configured such that one or more portions (e.g., regions, lengths, or sections) may be expandable relative to one or more other portions of the introducer sheath 1000. Accordingly, a first portion of the introducer sheath 1000 may be in the initial configuration (see, e.g., dₒ, FIG. 1), while a second portion of the introducer sheath 1000 is in the expanded configuration (e.g., with a diameter d₂) and/or while a third portion of the introducer sheath 1000 is in the recovered configuration (e.g., with a diameter d₁). Likewise, a portion of the introducer sheath 1000 may be in the expanded configuration (e.g., with a diameter d₂) while another portion of the introducer sheath 1000 is in the recovered configuration (e.g., with a diameter d₁). It is to be appreciated that the one or more portions (e.g., regions, lengths, or sections) of the introducer sheath 1000 may be of any length and thus are not limited to discrete length or discrete sections.

In various examples, the expanded diameter (e.g., d₂) is generally any diameter of the lumen 1006 greater than the initial diameter of the lumen 1006 that occurs in response to an outward force being exerted upon a given portion of the introducer sheath 1000. In various examples, as medical devices having profiles (e.g., diameters) in excess of the initial diameter of the lumen 1006 are advanced through the lumen 1006 of the introducer sheath 1000, such medical devices contact the introducer sheath 1000 (such as along an luminal wall of the lumen) and thereby exert an outward force upon the introducer sheath 1000 that causes the lumen 1006 of the introducer sheath 1000 to expand the region of the contact to accommodate the profile of the medical device.

In some examples, the introducer sheath 1000 is operable to have the lumen diameter expanded up three hundred (300) percent of the initial diameter while maintaining the ability of the introducer sheath 1000 to self-recover the diameter of the lumen to the post-delivery diameter. In some examples, the introducer sheath 1000 is operable to have the lumen diameter expanded from twenty-five (25) to one hundred percent (100), from one hundred (100) to one hundred twenty-five (125), from one hundred twenty-five (125) to two hundred (200), or from two hundred (200) to three hundred (300) percent of the initial diameter while maintaining the ability of the introducer sheath 1000 to self-recover the diameter of the lumen to the post-delivery diameter.

In various examples, the post-delivery diameter (e.g., d₁) generally refers to the diameter of the lumen 1006 after the introducer sheath 1000 has self-recovered the diameter of the lumen in response to a removal of the outward force (e.g., recovered configuration). That is, in various examples, the introducer sheath is configured to automatically recover to the post-delivery diameter without influence from any external forces. As mentioned herein, such a capability is based on the internal bias of the introducer sheath 1000, which is a result of the interaction between the support frame work (e.g., 1100 and/or 1300) and the covering 1200. The post-delivery diameter is therefore less than the expanded diameter.

In some examples, the post-delivery diameter and the initial diameter are the same diameter. In some examples, the post-delivery diameter is not more than ten (10) percent greater than the initial diameter (e.g., such as not more than 1, 2, 3, 4, 5, 6, 7, 8, or 9 percent greater than the initial diameter) and is less than the expanded diameter. In some examples, the post-delivery diameter is in a range of between 10 and 15 percent of the initial diameter. In some examples, the introducer sheath 1000 is configured to have a resting diameter (e.g., initial diameter or post-delivery diameter) that is less than the diameter of the anatomy in which the introducer sheath 1000 is located. For example, the combined internal forces of the support frame work (e.g., 1100 and/or 1300) and the covering 1200 are configured to maintain the introducer sheath 1000 at a diameter less than the diameter of the surrounding anatomy when no external forces (i.e., forces besides the internal forces of the support frame of covering) are applied to the introducer sheath 1000 (e.g., forces applied by a device positioned within the lumen 1006 of the introducer sheath 1000). Thus, the introducer sheath 1000 is configured to reduce or minimize contact with the surrounding tissue when not under the influence of external forces.

In some examples, the introducer sheath 1000 includes one or more lubricious liner layer to provide a lubricious interface that facilitates delivery of a medical device or apparatus through the introducer sheath 1000 with reduced friction between the medical device or apparatus to be delivered and the introducer sheath 1000. For example, the lubricious liner layer can be located at, define, or otherwise be included in an interior surface, such as the interior portion 1202, of the introducer sheath 1000, such that the lubrication provided by the lubricious liner layer reduces friction between the medical device and the interior portion 1202 during delivery. The lubricious liner layer has a smooth interior surface (e.g., a majority, substantial all, or an entirety of the surface) when the lumen 1006 of the introducer sheath 1000 is at the initial diameter and when the lumen 1006 is expanded to the expanded diameter. In some examples, the lubricious liner layer can be located around, define, or otherwise be included in an exterior surface, such as the exterior portion 1204, of the introducer sheath 1000, such that the introducer sheath 1000 can be advanced through another lumen, for example the inside of a separate catheter which couples to the introducer sheath 1000 during operation. In some examples, the introducer sheath 1000 has a smooth, continuous interior surface and a smooth, continuous exterior surface (e.g., a majority, substantial all, or an entirety of the surface) both when the sheath 1000 is at the initial diameter and when the sheath 1000 is expanded to the expanded diameter.

In some examples, the smoothness of the surfaces is maintained due to the elasticity of the sheath 1000. This can be contrasted to, for example, less elastic or inelastic designs that will fold, wrinkle, crease, pleat or otherwise be rendered non-smooth upon collapsing from the expanded to initial diameters. Various embodiments are particularly advantageous in that they incorporate a hydrogel coating or any other suitable lubricant, or lubricious surface treatment while also incorporating the elasticity required to transition between the initial and expanded diameters to remain smooth. Various examples of the lubricious liner layer materials and configurations are further explained herein.

The ability of the introducer sheath 1000 to self-recover provides that those regions of the introducer sheath 1000 not expanded as a result of any medical device(s) being present in such regions are operable to maintain a minimal profile within the vasculature, which provides for a reduced occlusion of the vessel, as well as a reduction in trauma to the vessel.

As mentioned above, the self-recoverability of the introducer sheath 1000 is based on a bias present in one or more of the support framework (e.g., 1100 and/or 1300) and the covering 1200. In various examples, such a bias is in the form of potential energy stored in one or more of the support framework (e.g., 1100 and/or 1300) and the covering 1200 that can be converted to kinetic energy to cause the introducer sheath 1000 to self-recover the diameter of the lumen 1006. In some examples, one or more of the support framework (1100 and/or 1300) and the covering 1200 are elastically deformable. Accordingly, in various examples, the introducer sheath 1000 is elastically deformable due, at least in part, to an elasticity of one or more of the support framework (e.g., 1100 and/or 1300) and the covering 1200 when elastically deformed.

In some examples, the covering 1200 is configured to stretch in accordance with the introducer sheath 1000 being expanded, where the covering 1200 stores potential energy when stretched (e.g., the covering 1200 is elastically deformed). In some examples, the covering 1200 is stretched or elastically deformed when the lumen 1006 of the introducer sheath 1000 is at the initial diameter. In some such examples, the covering 1200 is thus configured to store potential energy when the introducer sheath 1000 is in the initial configuration with the lumen 1006 at the initial diameter. In various examples, the potential energy of the covering 1200 is convertible to kinetic energy to help cause the introducer sheath 1000 to self-recover the diameter of the lumen 1006 to the post-delivery diameter after being expanded to a diameter greater than each of the initial and post-delivery diameters. In some examples, the potential energy is stored in the membrane material of the covering 1200. In some examples, the potential energy is additionally or alternatively stored in the reinforcing material of the covering 1200.

Additionally or alternatively, in some examples, the support framework (e.g., 1100 and/or 1300) is configured such that it stores potential energy in one or more of the above-referenced configurations of the introducer sheath 1000. For instance, in some examples, the support framework (1100 and/or 1300) stores potential energy when the lumen 1006 of the introducer sheath 1000 is expanded to a diameter greater than each of the initial and post-delivery diameters. In some such examples, the support framework may be shape set to a profile that the support framework adopts when the lumen 1006 of the introducer sheath 1000 is at the initial diameter (e.g., d₀). That is, in some examples, the support framework may be shape set to a profile that the support framework adopts when the introducer sheath 1000 is in the initial configuration. The support framework (1100 and/or 1300) may therefore include a shape memory material that is shape set to a configuration different from the configuration adopted by the support framework when the introducer sheath 1000 is expanded.

In other examples, however, the support framework (1100 and/or 1300) may be shape set to a configuration different from the configuration adopted by the support framework when the introducer sheath 1000 is in the initial configuration with the lumen 1006 at the initial diameter. In some such examples, the support framework (1100 and/or 1300) stores potential energy when the lumen 1006 of the introducer sheath 1000 is at one or more of the initial and post-delivery diameters. In some examples, the support framework may be shape set to a profile that the support framework adopts when the introducer sheath 1000 is in a configuration between the initial configuration and the expanded configuration (e.g., an intermediate configuration where the diameter of the lumen exceeds the initial diameter and where the diameter of the lumen is less than the expanded diameter). In various examples, the potential energy of the support framework is convertible to kinetic energy to help cause the introducer sheath 1000 to self-recover the diameter of the lumen 1006 to the post-delivery diameter after being expanded to a diameter greater than each of the initial and post-delivery diameters.

In those examples where the support framework (e.g., 1100 and/or 1300) is shape set to an intermediate configuration, it is to be appreciated that the support framework stores potential energy when the lumen of the introducer sheath 1000 has the initial diameter and when the introducer sheath 1000 is in the expanded configuration. It is thus to be appreciated that, in some examples, the introducer sheath 1000 may be configured such that each of the support framework (e.g., 1100 and/or 1300) and the covering 1200 stores potential energy when the introducer sheath 1000 is in the initial configuration. For instance, the support framework (e.g., 1100 and/or 1300) may be shape set to the above-referenced intermediate configuration, and the covering 1200 may be applied to the support framework such that the covering 1200 biases the introducer sheath 1000 toward a contracted configuration having a lumen diameter less than the initial diameter. Under such conditions, when the lumen 1006 of the introducer sheath 1000 has the initial diameter, the support framework (e.g., 1100 and/or 1300) is, and the covering 1200 is elastically tensioned.

The elastically radially contracted support framework (1100 and/or 1300) thus biases the introducer sheath 1000 radially outwardly (e.g., in a radially outwardly directed vector), and the covering 1200 thus biases the introducer sheath 1000 radially inwardly (e.g., in a radially inwardly directed vector). In some such examples, the radially outwardly directed bias of the support framework (1100 or 1300) and the radially inwardly directed bias of the covering 1200 are at equilibrium when the lumen 1006 of the introducer sheath 1000 has the initial diameter. Put differently, in some examples, the introducer sheath 1000 may be configured such that a first force associated with the potential energy of the covering 1200 and a second force associated with the potential energy of the support framework (e.g., 1100 and/or 1300) are at equilibrium with one another when the lumen 1006 of the introducer sheath 1000 has the initial diameter. In various examples, it is to be appreciated that equilibrium between such first and second forces also occurs when the lumen 1006 of the introducer sheath 1000 has the post-delivery diameter.

The various configurations of the introducer sheath 1000 described herein are such that the introducer sheath is configured to expand radially uniformly, such as relative to a longitudinal axis of the introducer sheath 1000. For example, unlike some conventional systems the introducer sheath 1000 of the present disclosure does not include a flap or fold or relief that operates to facilitate the expansion of the introducer sheath 1000. Instead as described herein, the introducer sheath 1000 of the present disclosure includes a framework (e.g., 1100 and/or 1300) and a covering 1200 that are arranged such that the covering 1200 stretches to accommodate expansion of the introducer sheath 1000. The configuration of the support framework is also such that the support framework expands radially uniformly. In other words, the introducer sheath 1000 does not include a fold, flap, relief, or other mechanism that facilitates an increase in diameter of the lumen of the introducer sheath 1000.

In various examples, in addition to expanding radially uniformly, the introducer sheath 1000 is configured such that the introducer sheath 1000 maintains a smooth exterior surface (e.g., a majority, substantial all, or an entirety of the surface) when the lumen is at the initial diameter, the expanded diameter (or expanded diameter), and the post-delivery diameter. For example, the exterior surface of the introducer sheath 1000 can remain smooth and wrinkle-free when radially expanded at about 50% of the initial diameter and greater, and the introducer sheath 1000 can expand and contract radially after deployment into a vessel in a body without the sheath 1000 infolding or otherwise deflecting inwardly into the inner lumen of the introducer sheath 1000. In some examples, the covering 1200 is applied to the support framework (e.g., 1100 and/or 1300) such that the covering 1200 is tensioned about (e.g., stores potential energy) the support framework in each of the introduction, expanded, and the post-delivery diameters, as mentioned above. At minimum, the covering 1200 is applied to the support framework in such that the covering 1200 is wrinkle-free in each of the introduction, expanded, and the post-delivery diameters, as mentioned above.

In various examples, the introducer sheath 1000 may be formed by providing a frame comprised of a plurality of strut elements arranged to form a tubular framework, and coupling the covering 1200 to the frame such that the frame and the covering 1200 define a sheath having a lumen extending therethrough. The frame corresponds with any of the support frameworks (e.g., 1100 and/or 1300) described herein. The covering 1200 corresponds with any of the coverings 1200 described herein.

In some examples, the method includes shape setting the frame to an initial configuration having an initial diameter. In some examples, the frame is shape set to an initial configuration where the lumen of the sheath is at the initial diameter (e.g., d₀) referred to herein. In some other examples, the frame is shape set to a configuration where the lumen of the sheath is at a diameter greater than the initial diameter. In such other examples, the covering 1200 may be coupled to the frame such that the frame is radially compressed to a contracted configuration such that potential energy is stored in the frame when the lumen of the sheath is at the initial diameter (e.g., d₀) referred to herein. Additionally or alternatively, coupling the covering 1200 to the frame may including coupling the covering 1200 to the frame such that the covering 1200 stores potential energy when the lumen of the sheath has the initial diameter (e.g., d₀) referred to herein. In some such examples, the covering 1200 may be stretched as it is coupled to the frame.

In various examples, the frame may be shape set to the initial configuration prior to or after the covering 1200 is coupled to the frame. In some examples, coupling the covering 1200 to the frame to define the sheath includes the use of a forming mandrel. In some such examples, the covering 1200 is disposed about a mandrel, and the frame is then positioned about the covering 1200. In some examples, the covering 1200 may be disposed about the covering 1200 on the mandrel such that a first portion of the covering 1200 is positioned between a first end and a second end of the frame and such that a second portion of the covering 1200 extends beyond one of the first and second ends of the frame. In such cases, the second portion of the covering 1200 may be trimmed. Alternatively, the second portion may be everted over the frame such that the frame is situated between the first and second portions of the covering 1200.

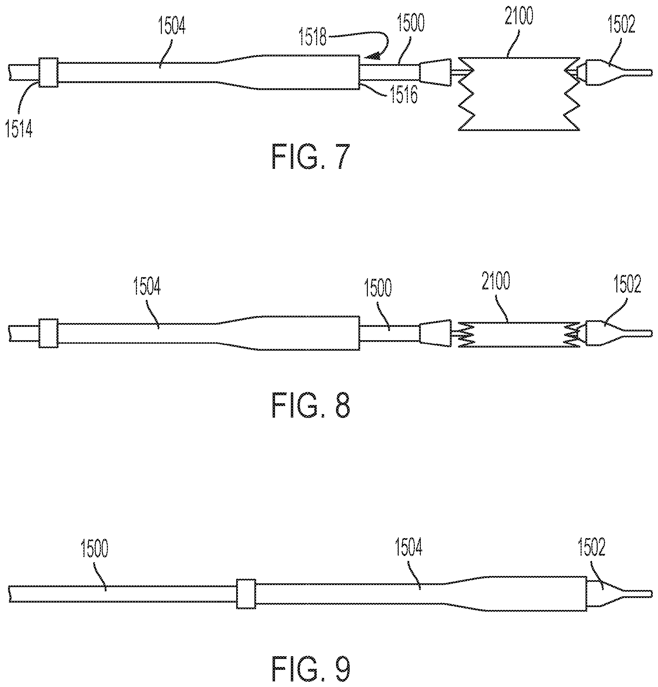

FIGs. 7 to 19 show a process of using the introducer sheath 1000 to deploy one or more medical devices 2100, which may include but are not limited to valve prostheses such as transcatheter mitral valve replacement (TMVR) prostheses and transcatheter aortic valve replacement (TAVR) prostheses as well as other types of suitable medical devices.

As shown in FIG. 7, the medical device 2100 is mounted on a delivery catheter 1500 proximate a distal end portion 1502 of the delivery catheter 1500. The delivery catheter 1500 as described herein can be formed using polymers such as silicone rubber, nylon, polyurethane, polyethylene terephthalate (PET), latex, and thermoplastic elastomers, among others. In some examples, the delivery catheter can also include a laser-cut hypotube formed using materials including but not limited to metals such as medical grade stainless steel, e.g. AISI 300 series stainless steel alloy, titanium, tantalum, alloys such as Elgiloy^{®} and Phynox^{®} spring alloys, MP35N^{®} alloy, and Nitinol nickel-titanium alloy, so that the mechanical properties of the catheter is improved, for example to achieve increased flexibility and/or better torque characteristics. Examples of suitable delivery catheters 1500 and associated implantable medical devices may be found in U.S. Patent App. Pub. 2019/0125534, "Transcatheter Deployment Systems and Associated Methods," filed Sept. 12, 2018.

In the illustration of FIG. 7, the medical device 2100 (e.g., a prosthetic heart valve) is shown in an expanded, or delivery configuration. An inline sheath dilator 1504 is placed on an exterior surface of the delivery catheter 1500 such that the delivery catheter 1500 passes through an inner lumen of the inline sheath dilator 1504. The inline sheath dilator 1504 has a proximal opening 1514, a distal opening 1516, and a dilator lumen 1518 extending therethrough. In some examples, the dilator lumen 1518 slidably receives the delivery catheter 1500 and the distal opening 1516 has a greater diameter than the proximal opening 1514.

FIG. 8 shows the medical device 2100 in a compacted or delivery configuration. In some examples, the medical device 2100 has an initial or compacted diameter ranging from 6 Fr to 16 Fr, for example. The diameter of the medical device 2100 in the compacted configuration may be approximately equal to or less than the diameter of the inner lumen of the inline sheath dilator 1504.

FIG. 9 shows the inline sheath dilator 1504 advanced over the compacted medical device 2100 toward the distal end portion 1502 so as to cover the medical device 2100, according to some methods of delivery. As shown, the inline sheath dilator 1504 is configured to engage the distal end portion 1502 (e.g., the distal end portion 1502 optionally has a diameter approximately equal to or greater than the inner diameter of the inline sheath dilator 1504.

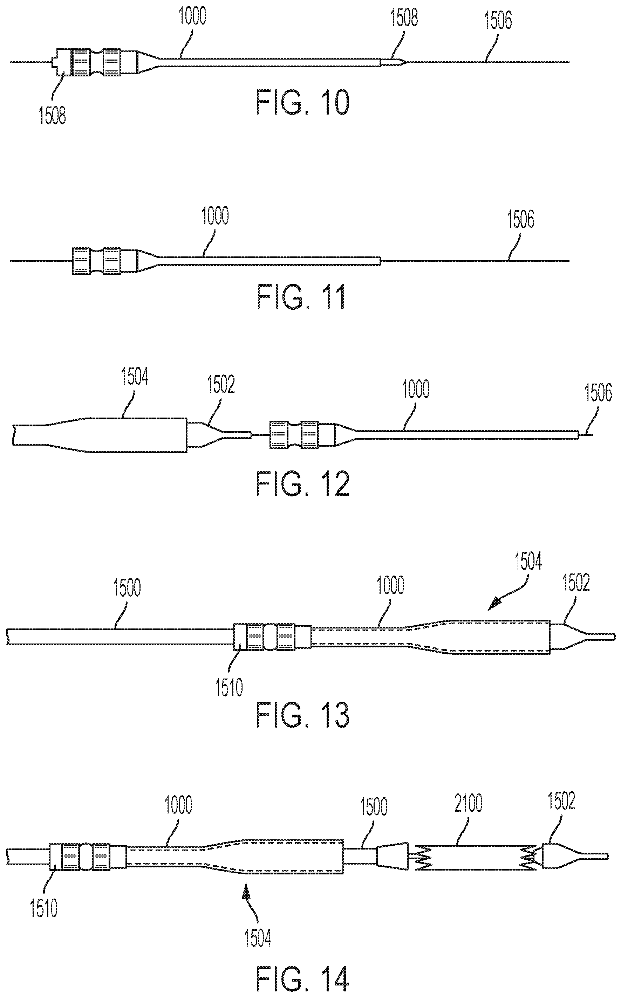

FIG. 10 shows the introducer sheath 1000 in an initial, unexpanded state. As shown in FIG. 10, a resilient dilator 1508 is inserted through an inner lumen of the sheath 1000 (e.g., to facilitate introduction of the sheath 1000 into a body of a patient). As shown, a guidewire 1506 is received through the sheath 1000. As shown, the dilator 1508 may be retracted to leave the sheath 1000 receiving the guidewire 1506.

Thereafter, as shown in FIG. 12, the delivery catheter 1500 with the inline sheath dilator 1504 as shown in FIG. 9 is advanced toward the introducer sheath 1000 over the guidewire 1506. For example, the delivery catheter 1500 is optionally passed through the auxiliary component 2000 (e.g., a hemostatic valve hub).

As shown in FIG. 13, the delivery catheter 1500 and the inline sheath dilator 1504 are inserted through the introducer sheath 1000 until a proximal mating portion 1510 of the inline sheath dilator 1504 comes into contact with a proximal portion of the introducer sheath 1000 (e.g., auxiliary component 2000). At this stage, the distal end portion 1502 of the delivery catheter 1500 protrudes from the introducer sheath 1000, and the inline sheath dilator 1504. In some examples, the proximal mating portion 1510 is coupled to the proximal portion of the introducer sheath 1000 (e.g., in a releasable engagement). As the inline sheath dilator 1504 is extended through the introducer sheath 1000 the inline sheath dilator expands the sheath 1000 to an expanded diameter greater than the initial diameter as the introducer sheath 1000 conforms to the shape and size of the inline sheath dilator 1504. For sake of visualization, in FIGs. 13, 14, 16, and 17, the location of the inline sheath dilator 1504 is shown with a dotted line.

As shown in FIG. 14, after the distal end of the delivery catheter 1500 is positioned proximate a desired delivery site in a body of a patient, the delivery catheter 1500 is able to be decoupled from the inline sheath dilator 1504 or otherwise extended from the distal end portion 1502 of the inline sheath dilator 1504 so that the delivery catheter 1500 can advance forward with the medical device 2100 in the compacted configuration. As shown in FIG. 14, the inline sheath dilator 1504 remains in place to maintain the introducer sheath 1000 in the expanded state.

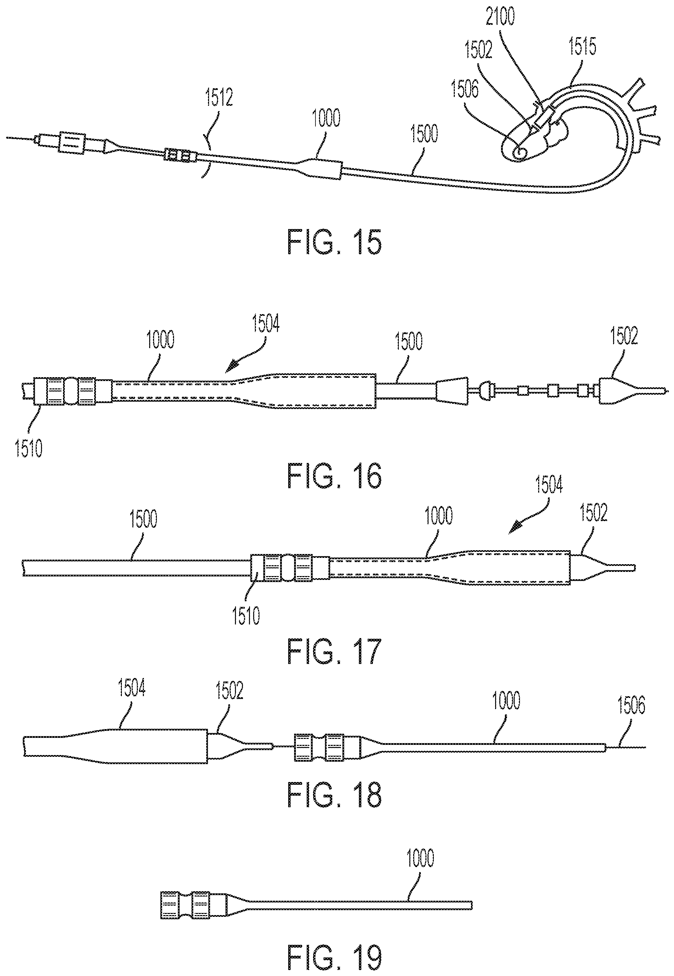

FIG. 15 shows one example of how the medical device 2100 is delivered to a desired location through a vasculature of a body. As shown, the introducer sheath 1000 is introduced through a percutaneous introduction site 1512, which may be an aperture that is surgically opened to insert the delivery catheter 1500 therethrough. The guidewire 1506 helps guide the distal end portion 1502 of the delivery catheter 1500 and thus the medical device 2100 to a desired location within the vasculature 1515 where the medical device 2100 may be deployed or other operation may be carried out.

FIG. 16 shows the delivery catheter 1500 after deploying the medical device 2100 within the vasculature 1515. In some examples, following device delivery, the delivery catheter 1500 is retracted so the distal end portion 1502 recouples with the inline sheath dilator 1504 as shown in FIG. 17. For example, such coupling may provide a smooth outer profile (e.g., at a majority, substantial all, or an entirety of the surface) for removal of the delivery catheter 1500 and inline sheath dilator 1504 from the body.

FIG. 18 shows the delivery catheter 1500, now coupled with the inline sheath dilator 1504, retracted further along the guidewire 1506 to be withdrawn in unison from the introducer sheath 1000. After the delivery catheter 1500 and the inline sheath dilator 1504 are retracted, the introducer sheath 1000 is withdrawn from the vasculature 1515 as shown in FIG. 19.

In the above examples, in order to facilitate effortless insertion of the inline sheath dilator 1504 through the introducer sheath 1000, a lubricious interface (e.g., one or more lubricious layers) can be disposed between the inline sheath dilator 1504 and the introducer sheath 1000 to reduce friction therebetween. In some examples, the lubricious interface is relatively non-distensible, or relatively inelastic.

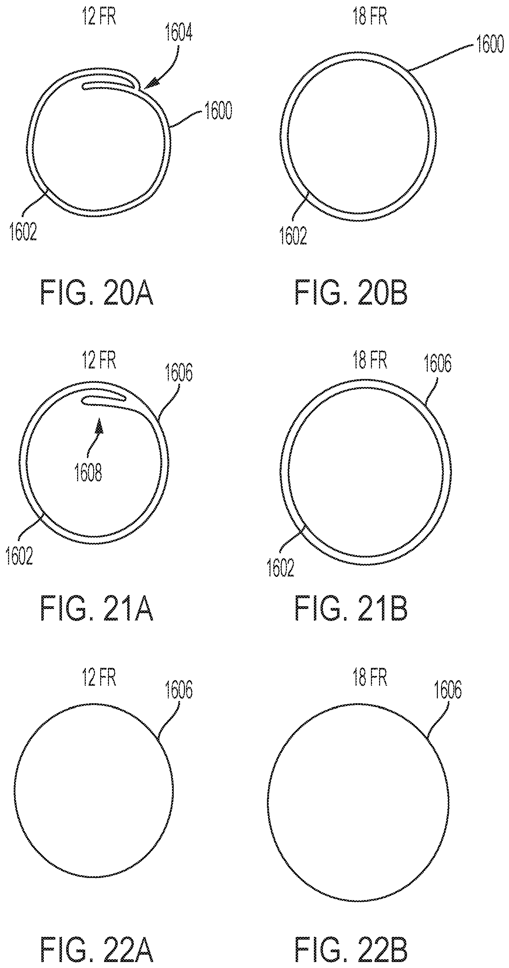

In the examples shown in FIGs. 20A, 20B, 21A, and 21B, the lubricious interface includes a tubular member defining an inelastic lubricious liner 1602 coupled to the introducer sheath 1000 which is another tubular member. The introducer sheath 1000 can be either inelastic (e.g., FIGs. 20A and 20B) or elastic (e.g., FIGs. 21A and 21B).

FIG. 20A shows an example where the introducer sheath 1000 includes an inelastic sheath 1600 with the inelastic lubricious liner 1602. As shown, the inelastic sheath 1600 is coupled with the inelastic lubricious liner 1602 so that the inelastic sheath 1600 and the inelastic lubricious liner 1602 both have a pleated configuration when collapsed to have an initial diameter. The pleat 1604 is formed by a portion of both the inelastic sheath 1600 and the inelastic lubricious liner 1602 that is folded over to overlap with another portion in the inelastic sheath 1600 and the inelastic lubricious liner 1602. The amount of overlap can be adjusted to achieve different diameters and expansion capabilities. In particular, when the inelastic sheath 1600 and the inelastic lubricious liner 1602 are expanded to an expanded diameter greater than the initial diameter, the pleat 1604 is released and the inelastic sheath 1600 and the inelastic lubricious liner 1602 become unpleated as shown in FIG. 20B. Although a single pleat is shown, any number of pleats may be incorporated as desired (e.g., two, three, four, six, twelve, and so forth).

FIG. 21A shows an example where the introducer sheath 1000 includes an elastic sheath 1606 configured to be elastically expanded and the inelastic lubricious liner 1602. As shown, a pleat 1608 is formed in the inelastic lubricious liner 1602 with a portion of the inelastic lubricious liner 1602 folded over another portion in an overlapped configuration. Although a single pleat is shown, any number of pleats may be incorporated as desired (e.g., two, three, four, six, twelve, and so forth). The elastic sheath 1606 remains in the unpleated configuration (e.g., and relatively wrinkle-free) regardless of whether the elastic sheath 1606 is at the initial diameter or the expanded diameter. On the other hand, as shown in FIG. 21B, the pleat 1608 of the inelastic lubricious liner 1602 is released and the inelastic lubricious liner 1602 becomes unpleated when the inelastic lubricious liner 1602 is expanded to the expanded diameter (e.g., corresponding to a state when the inline sheath dilator 1504 and/or delivery catheter 1500 is passed through the sheath.

In still other embodiments, the inline sheath dilator 1504 carries the lubricious interface. In some examples, an outer surface of the inline sheath dilator 1504 is comprised of a lubricious material including but not limited to PTFE, high-density polyethylene (HDPE), polyimide, or a thermoplastic with hydrophilic coating applied thereto. In some examples, an inner surface of the elastic sheath 1606 is comprised of a radially expandable elastomer including but not limited to silicone, polyurethane, thermoplastic elastomers (TPE), or thermoplastic vulcanizates (TPV).

As an example, FIG. 22A shows the sheath 1000 including the elastic sheath 1606 without an associated lubricious liner. In some examples, the lubricious layer, such as the inelastic lubricious liner 1602, is coupled with an outer surface of the inline sheath dilator 1504. In some examples, the lubricious layer is a hydrogel coating applied on the outer surface of the inline sheath dilator 1504 such that the elastic sheath 1606 can slide more easily along the outer surface of the inline sheath dilator 1504 as the inline sheath dilator 1504 is inserted into the elastic sheath 1606 to expand the elastic sheath 1606 to achieve the expanded diameter.

Although lubricous coatings or other types of liners and interfaces are contemplated, in some examples, the elastic sheath 1606 has an inner surface that is lubricious so as to not require any additional or supplemental lubricious liner within the elastic sheath 1606. For example, the elastic sheath may include an inner surface formed of polymers including but not limited to PTFE or ePTFE imbibed with a lubricious hydrogel. In one example, the lubricious hydrogel is an organic compound including but not limited to polyol, polyvinylpyrrolidone (PVP), isocyanate, or any other suitable lubricant. Incorporation of a lubricious inner surface, or a lubricious liner layer, can help reduce friction between the outer surface of the inline sheath dilator 1504 and the inner surface of the elastic sheath 1606. In some examples, the lubricious liner layer provides a smooth interior surface when the lumen of the elastic sheath 1606 is at the initial, smaller diameter and also provides a smooth interior surface when the lumen of the elastic sheath 1606 is at the expanded, larger diameter. Various materials may be implemented to achieve smooth configurations (e.g., at a majority, substantial all, or an entirety of the surface) at both the initial, smaller diameter and the expanded, larger diameter, such as the retracted membrane and retracted membrane composites previously described.

Incorporation of expandable, lubricious liners provide the ability to provide a continuous, smooth surface (e.g., a majority, substantial all, or an entirety of the surface) without the need for incorporating features for splitting, unfolding, releasably pleating, or otherwise accommodating relatively inextensible materials into the liner to permit expansion from an initial diameter to a larger diameter.

In some examples, when the inner surface of the elastic sheath 1606 is lubricious, an outer surface of the elastic sheath 1606 is not lubricious. In some examples, the elastic sheath 1606 is formed by fusing or attaching two or more layers of different polymeric membrane together, where one or more outer membrane layers define the properties of the outer surface of the elastic sheath 1606 while one or more inner layers define the properties of the inner surface of the elastic sheath 1606. In some examples, the outer layer(s) are a polymer that is not imbibed with a lubricious hydrogel material (e.g., relatively free of a lubricious component), whereas the inner layer is a polymer that is imbibed with the lubricious hydrogel material (or other lubricious component) such that the elastic sheath 1606 has a lubricious inner surface and a non-lubricious outer surface.

### EXAMPLE

An expandable introducer sheath was manufactured according to the following method. An unimbibed polymeric tape was cigarette wrapped about a cylindrical mandrel twenty times (e.g., longitudinally wrapped with the transverse direction of the tape being perpendicular to the length of the mandrel). The mandrel and tape were then heated for a period of approximately 10 minutes. After cooling, fifteen layers of a polymeric tape imbibed with a reinforcing material were cigarette wrapped over the existing twenty layers of unimbibed polymeric tape such that the reinforcing material faced the mandrel. The mandrel and tape were then heated for approximately five minutes. A support framework consistent with the disclosure herein was then disposed about the wrapped polymeric tapes. An excess length of the polymeric tapes extending beyond an end of the support framework was then everted over an exterior of the support framework. Another polymeric tape was then wrapped about a portion of the construct to maintain relative positionings of the tapes and the support framework. The construct was then heated for a period of 5 minutes. After cooling, the construct was removed from the mandrel.

Numerous characteristics and advantages have been set forth in the preceding description, including various alternatives together with details of the structure and function of the devices and/or methods. The disclosure is intended as illustrative only and as such is not intended to be exhaustive.

## Claims

1. A medical apparatus (10), comprising:
a frame (1100) comprised of a plurality of strut elements (1108) arranged to form a tubular framework, and
a covering (1200) extending along a portion of the frame (1100) such that the frame (1100) and the covering (1200) define a sheath (1000) having a lumen (1006) extending therethrough, wherein the strut elements (1108) of the frame (1100) interact with one another to provide the sheath (1000) with axial compressive resistance and hoop strength, the lumen (1006) having a length and the sheath (1000) being radially expandable such that a diameter of the lumen (1006) is operable to be expanded from an initial diameter to an expanded diameter greater than the initial diameter to accommodate the passage of one or more devices therethrough, wherein the sheath (1000) is further configured to self-recover the diameter of the lumen (1006) along the length of the sheath (1000) to a post-delivery diameter that is less than the expanded diameter in response to the one or more devices being removed from the lumen (1006).

2. The medical apparatus (10) of claim 1, wherein the covering (1200) is configured to stretch such that the sheath (1000) is radially expandable, and wherein the covering (1200) stores potential energy when the lumen (1006) of the sheath (1000) is expanded to the expanded diameter, the potential energy of the covering (1200) being convertible to kinetic energy to cause the sheath (1000) to self-recover the diameter of the lumen (1006) to the post-delivery diameter.

3. The medical apparatus (10) of any of the preceding claims, wherein the frame (1100) is configured to store potential energy when the sheath (1000) is expanded to the expanded diameter, the potential energy of the frame (1100) being convertible to kinetic energy to cause the sheath (1000) to self-recover the diameter of the lumen (1006) to the post-delivery diameter.

4. The medical apparatus (10) of any of claims 1 to 3, wherein the frame (1100) is shape set to an initial configuration where the diameter of the lumen (1006) of the sheath (1000) is greater than the initial diameter such that the frame (1100) stores potential energy when the lumen (1006) of the sheath (1000) has the initial diameter.

5. The medical apparatus (10) of any of the preceding claims wherein the post-delivery diameter is not more than 115% of the initial diameter.

6. The medical apparatus (10) of any of the preceding claims, wherein the sheath (1000) is configured such that the sheath (1000) has a smooth exterior surface when the sheath (1000) self-recovers the diameter of the lumen (1006) to the post-delivery diameter; or
wherein the covering (1200) forms a lubricious impermeable layer of the sheath (1000).

7. The medical apparatus (10) of any of the preceding claims, wherein a plurality of discrete zones are defined along an axial length of the sheath (1000), and wherein the sheath (1000) is configured to be progressively expanded and self-recovered such that the diameter of the lumen (1006) at a first zone of the plurality of discrete zones is expandable relative to the diameter of the lumen (1006) a second zone of the plurality of discrete zones, and such that the diameter of the lumen (1006) at the first zone is self-recoverable to the post-delivery diameter.

8. The medical apparatus (10) of any of the preceding claims, wherein the diameter of the lumen (1006) is configured to be expandable to the expanded diameter at first cross section along an axial length of the sheath (1000) without causing expansion of the diameter of the lumen (1006) from the initial diameter at a second cross section along the axial length of the sheath (1000), and wherein the diameter of the lumen (1006) configured to be self-recoverable to the post-delivery diameter at the first cross section without causing a reduction of the diameter of the lumen (1006) at the second cross section.

9. The medical apparatus (10) of claim 1, wherein the lumen (1006) is configured to expand to accommodate medical devices as they are advanced distally through the lumen (1006).

## Patentansprüche

1. Medizinische Vorrichtung (10), umfassend:
einen Rahmen (1100), der aus einer Vielzahl von Strebenelementen (1108) besteht, die angeordnet sind, um ein rohrförmiges Rahmengerüst zu bilden; und
eine Abdeckung (1200), die sich entlang eines Abschnitts des Rahmens (1100) erstreckt, sodass der Rahmen (1100) und die Abdeckung (1200) eine Hülle (1000) definieren, die ein Lumen (1006) aufweist, das sich hindurch erstreckt, wobei die Strebenelemente (1108) des Rahmens (1100) miteinander interagieren, um die Hülle (1000) mit axialer Druckfestigkeit und Umfangsfestigkeit zu versehen, wobei das Lumen (1006) eine Länge aufweist und die Hülle (1000) radial dehnbar ist, sodass ein Durchmesser des Lumens (1006) betreibbar ist, um von einem Anfangsdurchmesser auf einen gedehnten Durchmesser, der größer als der Anfangsdurchmesser ist, gedehnt zu werden, um den Durchgang einer oder mehrerer Vorrichtungen hindurch zu ermöglichen, wobei die Hülle (1000) ferner dazu konfiguriert ist, den Durchmesser des Lumens (1006) entlang der Länge der Hülle (1000) auf einen Durchmesser nach der Zuführung, der weniger ist als der gedehnte Durchmesser, als Reaktion darauf von selbst zurückführen, dass die eine oder mehrere Vorrichtungen aus dem Lumen (1006) entfernt werden.

2. Medizinische Vorrichtung (10) nach Anspruch 1, wobei die Abdeckung (1200) dazu konfiguriert ist, sich derart zu dehnen, dass die Hülle (1000) radial dehnbar ist, und wobei die Abdeckung (1200) potenzielle Energie speichert, wenn das Lumen (1006) der Hülle (1000) auf den gedehnten Durchmesser gedehnt wird, wobei die potenzielle Energie der Abdeckung (1200) in kinetische Energie umwandelbar ist, um zu bewirken, dass die Hülle (1000) den Durchmesser des Lumens (1006) von selbst auf den Durchmesser nach der Zuführung zurückführt.

3. Medizinische Vorrichtung (10) nach einem der vorhergehenden Ansprüche, wobei der Rahmen (1100) dazu konfiguriert ist, potenzielle Energie zu speichern, wenn die Hülle (1000) auf den gedehnten Durchmesser gedehnt wird, wobei die potenzielle Energie des Rahmens (1100) in kinetische Energie umwandelbar ist, um zu bewirken, dass die Hülle (1000) den Durchmesser des Lumens (1006) von selbst auf den Durchmesser nach der Zuführung zurückführt.

4. Medizinische Vorrichtung (10) nach einem der Ansprüche 1 bis 3, wobei der Rahmen (1100) in seiner Form auf eine Anfangskonfiguration eingestellt ist, bei der der Durchmesser des Lumens (1006) der Hülle (1000) größer als der Anfangsdurchmesser ist, sodass der Rahmen (1100) potenzielle Energie speichert, wenn das Lumen (1006) der Hülle (1000) den Anfangsdurchmesser aufweist.

5. Medizinische Vorrichtung (10) nach einem der vorhergehenden Ansprüche, wobei der Durchmesser nach der Zuführung nicht mehr als 115 % des Anfangsdurchmessers beträgt.

6. Medizinische Vorrichtung (10) nach einem der vorhergehenden Ansprüche, wobei die Hülle (1000) derart konfiguriert ist, dass die Hülle (1000) eine glatte Außenfläche aufweist, wenn die Hülle (1000) den Durchmesser des Lumens (1006) von selbst auf den Durchmesser nach der Zuführung zurückführt; oder
wobei die Abdeckung (1200) eine gleitfähige undurchlässige Schicht der Hülle (1000) bildet.

7. Medizinische Vorrichtung (10) nach einem der vorhergehenden Ansprüche, wobei eine Vielzahl von diskreten Zonen entlang einer axialen Länge der Hülle (1000) definiert sind und wobei die Hülle (1000) dazu konfiguriert ist, progressiv gedehnt und von selbst zurückgeführt zu werden, sodass der Durchmesser des Lumens (1006) an einer ersten Zone der Vielzahl von diskreten Zonen relativ zu dem Durchmesser des Lumens (1006) an einer zweiten Zone der Vielzahl von diskreten Zonen dehnbar ist und sodass der Durchmesser des Lumens (1006) an der ersten Zone von selbst auf den Durchmesser nach der Zuführung zurückführbar ist.

8. Medizinische Vorrichtung (10) nach einem der vorhergehenden Ansprüche, wobei der Durchmesser des Lumens (1006) dazu konfiguriert ist, an einem ersten Querschnitt entlang einer axialen Länge der Hülle (1000) auf den gedehnten Durchmesser dehnbar zu sein, ohne eine Dehnung des Durchmessers des Lumens (1006) von dem Anfangsdurchmesser an einem zweiten Querschnitt entlang der axialen Länge der Hülle (1000) zu bewirken, und wobei der Durchmesser des Lumens (1006) dazu konfiguriert ist, an dem ersten Querschnitt von selbst auf den Durchmesser nach der Zuführung zurückgeführt zu werden, ohne eine Verringerung des Durchmessers des Lumens (1006) an dem zweiten Querschnitt zu bewirken.

9. Medizinische Vorrichtung (10) nach Anspruch 1, wobei das Lumen (1006) dazu konfiguriert ist, sich zu dehnen, um medizinische Vorrichtungen aufzunehmen, da sie distal durch das Lumen (1006) vorgeschoben werden.

## Revendications

1. Appareil médical (10), comprenant :
un cadre (1100) constitué d'une pluralité d'éléments d'entretoises (1108) agencés de manière à former une structure tubulaire ; et
un revêtement (1200) s'étendant le long d'une partie du cadre (1100) de sorte que le cadre (1100) et le revêtement (1200) définissent une gaine (1000) possédant une lumière (1006) s'étendant à travers celle-ci, lesdits éléments d'entretoise (1108) du cadre (1100) interagissant les uns avec les autres de manière à fournir à la gaine (1000) une résistance à la compression axiale et une résistance périphérique, la lumière (1006) présentant une longueur et la gaine (1000) étant radialement extensible de sorte qu'un diamètre de la lumière (1006) puisse être étendu d'un diamètre initial à un diamètre élargi supérieur au diamètre initial de manière à permettre le passage d'un ou plusieurs dispositifs à travers celle-ci, ladite gaine (1000) étant en outre conçue pour récupérer d'elle-même le diamètre de la lumière (1006) le long de la longueur de la gaine (1000) jusqu'au diamètre après administration qui est inférieur au diamètre élargi en réponse au retrait desdits un ou plusieurs dispositifs de la lumière (1006).

2. Appareil médical (10) selon la revendication 1, ledit revêtement (1200) étant conçu pour s'étirer de sorte que la gaine (1000) soit radialement extensible, et ledit revêtement (1200) stockant de l'énergie potentielle lorsque la lumière (1006) de la gaine (1000) est élargie jusqu'au diamètre élargi, ladite énergie potentielle du revêtement (1200) pouvant être convertie en énergie cinétique pour amener la gaine (1000) à récupérer d'elle-même le diamètre de la lumière (1006) jusqu'au diamètre après administration.

3. Appareil médical (10) selon l'une quelconque des revendications précédentes, ledit cadre (1100) étant conçu pour stocker de l'énergie potentielle lorsque la gaine (1000) est élargie jusqu'au diamètre élargi, ladite énergie potentielle du cadre (1100) pouvant être convertie en énergie cinétique pour amener la gaine (1000) à récupérer d'elle-même le diamètre de la lumière (1006) jusqu'au diamètre après administration.

4. Appareil médical (10) selon l'une quelconque des revendications 1 à 3, ledit cadre (1100) étant de forme définie à une configuration initiale dans laquelle le diamètre de la lumière (1006) de la gaine (1000) est supérieur au diamètre initial de sorte que le cadre (1100) stocke de l'énergie potentielle lorsque la lumière (1006) de la gaine (1000) présente le diamètre initial.

5. Appareil médical (10) selon l'une quelconque des revendications précédentes, ledit diamètre après administration n'étant pas supérieur à 115 % du diamètre initial.

6. Appareil médical (10) selon l'une quelconque des revendications précédentes, ladite gaine (1000) étant conçue de sorte que la gaine (1000) présente une surface externe lisse lorsque la gaine (1000) récupère d'elle-même le diamètre de la lumière (1006) jusqu'au diamètre après administration ; ou
ledit revêtement (1200) formant une couche imperméable lubrifiante de la gaine (1000).

7. Appareil médical (10) selon l'une quelconque des revendications précédentes, une pluralité de zones discrètes étant définies le long de la longueur axiale de la gaine (1000), et ladite gaine (1000) étant conçue pour être progressivement élargie et récupérée d'elle-même de sorte que le diamètre de la lumière (1006) au niveau d'une première zone de la pluralité de zones discrètes soit extensible par rapport au diamètre de la lumière (1006) d'une seconde zone de la pluralité de zones discrètes, et de sorte que le diamètre de la lumière (1006) au niveau de la première zone puisse être récupéré de lui-même jusqu'au diamètre après administration.

8. Appareil médical (10) selon l'une quelconque des revendications précédentes, ledit diamètre de la lumière (1006) étant conçu pour être extensible jusqu'au diamètre élargi au niveau d'une première section transversale le long de la longueur axiale de la gaine (1000) sans provoquer l'élargissement du diamètre de la lumière (1006) à partir du diamètre initial au niveau d'une seconde section transversale le long de la longueur axiale de la gaine (1000), et ledit diamètre de la lumière (1006) étant conçu pour être récupéré de lui-même jusqu'au diamètre après administration au niveau de la première section transversale sans provoquer de réduction du diamètre de la lumière (1006) au niveau de la seconde section transversale.

9. Appareil médical (10) selon la revendication 1, ladite lumière (1006) étant conçue pour s'élargir de manière à s'adapter aux dispositifs médicaux lorsqu'ils sont avancés distalement à travers la lumière (1006).
